# EUROPEAN PATENT APPLICATION

(11) **EP 2 251 042 A2**
(43) Date of publication of application: **17.11.2010**
(21) Application number: 10171365.9
(22) Date of filing: 20.09.2007
(51) Int. Cl.: A61K 49/00, A61P 35/02, A61K 31/00, A61K 31/506

(54) **Method of optimizing the treatment of philadelphia-positive leukemia with abl tyrosine kinase inhibitors**

(30) Priority: 22.09.2006 US 826622 P; 05.10.2006 US 828278 P
(62) Divisional of application: 07842842.2
(71) Applicant: Novartis AG, 4056 Basel (CH)
(72) Inventor: Gathmann, Insa, 4057, Basel (CH); Mahon, Francois-Xavier, 33000, Bordeaux (FR); Molimard, Mathieu, 33076, Bordeaux (FR); Picard, Stéphane, 33076, Bordeaux (FR); Wang, Yanfeng, Florham Park, NJ 07932 (US)
(74) Representative: Roth, Peter Richard

(57) **Abstract**

The present invention provides a method of treating Philadelphia positive (Ph+) leukemia, such as Philadelphia chromosome positive acute lymphoblastic leukemia (Ph+ ALL) or chronic myeloid leukemia (CML), in a human patient population comprising the steps of
(a) administering a predetermined fixed amount of a Bcr-Abl tyrosine kinase inhibitor, such as Imatinib, or a pharmaceutically acceptable salt thereof to human patients suffering from a Ph+ leukemia,
(b) collecting at least one blood sample from said patients,
(c) determining the plasma trough level (Cmin) of the Bcr-Abl tyrosine kinase inhibitor or of a metabolite thereof as well as the MMR rates,
(d) assessing a discrimination potential of trough plasma concentrations for MMR and identifying a Cmin threshold for optimal sensitivity and specificity and
(e) adjusting the dose of the inhibitor of the Bcr-Abl tyrosine kinase or a pharmaceutically acceptable salt thereof applied to the individual patients from said patient population and, optionally, future patients suffering from a Ph+ leukemia in a manner that a Cmin is achieved in each single patient equal to or higher than the Cmin threshold obtained under step (d).

## Description

The present invention relates to a method of treating Philadelphia-positive leukemia (Ph+ leukemia) in a human patient population. In a particular aspect, the present invention relates to a method of treating chronic myeloid leukemia (CML) in a human patient population.

In CML a reciprocally balanced chromosomal translocation in hematopoietic stem cells (HSCs) produces the BCR-ABL hybrid gene. The latter encodes the oncogenic Bcr-Abl fusion protein. Whereas ABL encodes a tightly regulated protein tyrosine kinase, which plays a fundamental role in regulating cell proliferation, adherence and apoptosis, the BCR-ABL fusion gene encodes as constitutively activated kinase, which transforms HSCs to produce a phenotype exhibiting deregulated clonal proliferation, reduced capacity to adhere to the bone marrow stroma and a reduces apoptotic response to mutagenic stimuli, which enable it to accumulate progressively more malignant transformations. The resulting granulocytes fail to develop into mature lymphocytes and are released into the circulation, leading to a deficiency in the mature cells and increased susceptibility to infection. ATP-competitive inhibitors of Bcr-Abl have been described which prevent the kinase from activating mitogenic and anti-apoptotic pathways (e.g. P-3 kinase and STAT5), leading to the death of the BCR-ABL phenotype cells and thereby providing an effective therapy against CML.

In May 2001 the mesylate salt of N-{5-[4-(4-methyl-piperazino-methyl)-benzoylamido]-2-methylphenyl}-4-(3-pyridyl)-2-pyrimidine-amine (Imatinib mesylate, STI571, Glivec®) was approved by the FDA for the treatment of CML in patients who had failed to benefit from IFN-alpha therapy. Already in June 2000, the first CML patients were enrolled in the International Randomized Study of Interferon and STI571 (IRIS). This ambitious phase 3 trial was unique both in size and scope. The IRIS investigators recruited over 1000 patients in 16 countries to conduct a head-to-head comparison between Glivec and interferon-alpha (S.G. O'Brien, F. Guilhot, R.A. Larson, et al, N. Engl. J. Med. 2003, 348: 994-1004). Imatinib at a dose of 400 mg daily has shown superior efficacy to IFN+Ara-C for newly diagnosed patients with CML in chronic phase (CML-CP). Recently, five year IRIS follow-up data indicated an estimated cumulative rate of complete cytogenetic response (CCR) of 87% among patients who received first-line imatinib and an overall survival of 89% (Druker BJ, Guilhot F, O'Brien SG, et al on behalf of the IRIS Investigators. Five-Year Follow-up of Imatinib Therapy for Newly Diagnosed Myeloid Leukemia in Chronic-Phase Shows Sustained Responses and High Overall Survival. New Eng J Med 2006; 355:2408-17). Notably, no patient who achieved CCR and major molecular response (MMR) within 18 months of initiation of therapy has progressed to accelerated or blast phase at 60 months.

Still, variable responses to Imatinib mesylate in the treatment of chronic CML are incompletely understood. Previous studies focused on cellular mechanisms of resistance to Imatinib. Whereas pharmacokinetic monitoring is widely used in different medical specialities, such as neurology, cardiology, and psychiatry, it is has rarely been applied in clinical oncology practice. Pharmacokinetic studies in CML patients being treated with Imatinib mesylate showed that plasma trough concentrations of Imatinib are correlated with Imatinib mesylate dose, whereas body weight or body surface are of minor importance. Peng et al. determined trough plasma concentration of Imatinib and adjusted the Imatinib regimen according plasma concentration parameters (Peng B., Hayes M., Resta, D. et al, J. Clin. Oncol. 2004, 22, 935-942). Mahon et al. (Blood. 106(11, Part 1). Nov. 16 2005. 565A) measured blood concentration of Imatinib in support of the treatment regimen.

The present invention relates to a method for minimizing or avoiding the issues of tolerability, lack of efficacy and the risk of relapse in human CML patients being treated with a Bcr-Abl tyrosine kinase inhibitor. Based on the analysis of a study conducted at the University of Bordeaux and the IRIS study data correlating pharmacokinetic data with cytogenetic and molecular response in newly diagnosed patients with CML in chronic phase (CML-CP) it was now surprisingly found that the treatment of CML using a Bcr-Abl tyrosine kinase inhibitor can be optimized by adjusting the dose of the Bcr-Abl tyrosine kinase inhibitor applied to an individual patient in a manner that a specific minimum plasma trough level (Cmin) is achieved in each single patient. An individual adjustment for each patient is required in view of the high patient intervariability of the Cmin values upon administration of the same dose of the Bcr-Abl tyrosine kinase inhibitor to each patient as observed in the IRIS study. The present invention provides for the first time an individualized treatment schedule for single CML patients based on a Cmin lower threshold which was shown to be correlated with an increased chance of survival.

CML belongs to the group of Ph+ leukemia. The results obtained with the CML patient population described herein can be transferred directly to the whole group of Ph+ leukemias. The reason for that is that the characterizing feature of Ph+ leukemias is the existence of the Philadelphia chromosome causing the Bcr-Abl fusion protein. The latter protein is the target of all Bcr-Abl inhibitors.

The abbreviation "Ph+ ALL" as used herein denotes Philadelphia chromosome positive acute lymphoblastic leukemia.

The term "major molecular response (MMR)" as used herein means a 3 logarithm reduction in *BCR-ABL* transcripts, quantified from peripheral blood using real-time quantitative reverse-transcriptase polymerase chain reaction, preferably after 12 months of therapy, e.g. 12 months Imatinib mesylate therapy.

The term "complete cytogenic response (CCR)" as used herein means 0 % Philadelphia-chromosome positive metaphases among at least 20 or 25 cells in metaphase in the bone marrow aspirate (Colombat M, Fort MP, Chollet C, et al. Molecular remission in chronic myeloid leukemia patients with sustained complete cytogenetic remission after imatinib mesylate treatment. Haematologica 2006;91:162-8.).

The term "method of treatment" as used herein relates also to a method of prevention of the diseases mentioned herein, i.e. the prophylactic administration of a pharmaceutical composition comprising a Bcr-Abl tyrosine kinase inhibitor to healthy patients to prevent the development of the diseases mentioned herein.

The terms "adjusting the dose" and "the dose of ... is adjusted" as used herein preferably denote that the dose referred to is increased or decreased. In a broader sense of the invention, the terms "adjusting the dose" and the "dose of ... is adjusted" encompass a situation wherein the dose remains unchanged.

The term "Bcr-Abl tyrosine kinase inhibitor" as used herein relates to organic compounds that show inhibition of c-Abl or Bcr-Abl from lysates of transfected cells with an IC50 value below 0.1 µM in *in vitro* kinase assays performed on immunoprecipitates in an assay as described by B.J. Druker et al in Nat. Med. 1996, 2, 561-566.

Hence, in a broader sense, the present invention relates to a method of treating Ph+ leukemia, such as CML or Ph+ ALL, in a human patient population comprising the steps of
(a) administering a predetermined fixed amount of a Bcr-Abl tyrosine kinase inhibitor or a pharmaceutically acceptable salt thereof to human patients suffering from a Ph+ leukemia,
(b) collecting at least one blood sample from said patients,
(c) determining the plasma trough level (Cmin) of the Bcr-Abl tyrosine kinase inhibitor or of a metabolite thereof as well as the MMR rates,
(d) assessing a discrimination potential of trough plasma concentrations for MMR and identifying a Cmin threshold for optimal sensitivity and specificity, e.g. by Receiver Operating Characteristic (ROC) curve analysis, and
(e) adjusting the dose of the inhibitor of the Bcr-Abl tyrosine kinase or a pharmaceutically acceptable salt thereof applied to the individual patients from said patient population and, optionally, future patients suffering from a Ph+ leukemia in a manner that a Cmin is achieved in each single patient equal to or higher than the Cmin threshold obtained under step (d).

More specifically, the present invention pertains to a method of treating CML in a human patient population comprising the steps of
(a) administering a predetermined fixed amount of a Bcr-Abl tyrosine kinase inhibitor or a pharmaceutically acceptable salt thereof to human CML patients in need thereof,
(b) collecting at least one blood sample from said patients,
(c) determining Cmin of the Bcr-Abl tyrosine kinase inhibitor or of a metabolite thereof as well as the MMR rates,
(d) assessing a discrimination potential of trough plasma concentrations for MMR and identifying a Cmin threshold for optimal sensitivity and specificity, e.g. by ROC curve analysis, and
(e) adjusting the dose of the inhibitor of the Bcr-Abl tyrosine kinase or a pharmaceutically acceptable salt thereof applied to the individual patients from said patient population and, optionally, future CML patients in a manner that a Cmin is achieved in each single patient equal to or higher than the Cmin threshold obtained under step (d).

By the methodology described above, it was found that the Cmin threshold for the Bcr-Abl tyrosine kinase inhibitor Imatinib should be about 800 ng/mL, more preferably about 1000 ng/mL. The upper limit of the plasma level corresponds to the level closely below the blood level causing dose limiting toxicities (DLT) in an individual patient. Typically, the upper range observed is about 3500 ng/mL, sometimes about 3000 ng/mL.

Hence, in yet a further aspect, the present invention pertains to a method of treating a Ph+ leukemia, especially CML or Ph+ ALL, in a human patient comprising the steps of
(a) administering a predetermined fixed amount of Imatinib or a pharmaceutically acceptable salt thereof, e.g. an oral daily dose 400 mg or 800 mg of the mono-mesylate salt of Imatinib, to the human patient suffering from a Ph+ leukemia,
(b) collecting at least one blood sample from said patient, e.g. within the first 12 months of treatment,
(c) determining Cmin of Imatinib, and
(d) adjusting the dose of Imatinib or a pharmaceutically acceptable salt thereof in a manner that a Cmin of at least 800 ng/mL, especially between about 800 and about 3500 ng/mL, preferably a Cmin between 1000 and about 3000 ng/mL, of Imatinib is achieved in said patient.

In a broader sense, the present invention provides a method of treating a Ph+ leukemia, especially CML or Ph+ ALL, in a human patient wherein the dose of Imatinib or a pharmaceutically acceptable salt thereof is adjusted in a manner that a Cmin of at least 800 ng/mL, especially between about 800 and about 3500 ng/mL, preferably a Cmin between 1000 and about 3000 ng/mL, of Imatinib is maintained in said patient.

More specifically, the present invention relates to a method of treating CML in a human patient comprising the steps of
(a) administering a predetermined fixed amount of Imatinib or a pharmaceutically acceptable salt thereof to the human CML patient in need thereof,
(b) collecting at least one blood sample from said patient, e.g. within the first 12 months, especially the first 3 months, more especially the first 30 days, of treatment,
(c) determining the plasma trough level (Cmin) of Imatinib, and
(d) adjusting the dose of Imatinib or a pharmaceutically acceptable salt thereof in a manner that a Cmin of at least 800 ng/mL, especially between about 800 and about 3500 ng/mL of Imatinib is achieved in said patient.

In the latter method the dose of the pharmaceutically acceptable salt of Imatinib is adjusted preferably in a manner that a Cmin between about 1000 and about 3000 ng/mL of Imatinib is achieved in said patient, more preferably a Cmin of about 1000 ng/mL.

Imatinib undergoes metabolism through the cytochrome P450 system, CYP3A4 is the major isoenzyme responsible for imatinib metabolism, although CYP1A2, CYP2D6, CYP2C9, and CYP2C19 also contribute to a minor extent. One major metabolite, N-{5-[4-(piperazino-methyl)-benzoylamido]-2-methylphenyl}-4-(3-pyridyl)-2-pyrimidine-amine (CGP74588), is observed in blood which has a similar biological activity as Imatinib and represents approximately 20% of the parent drug plasma level in patients. Due to intrinsic variability of CYP enzyme activity (Wilkinson GR, J Pharmacokinet Biopharm. 1996; 24:475-90.) high inter-patient variability has been reported in imatinib exposure in CML patients (Peng BM Hayes M, Resta D, et al. J Clin Oncol. 2004;22:935-42). Drugs that inhibit or induce the CYP3A4 isozyme have been shown to influence imatinib pharmacokinetics (Bolton AE, Peng B, Hubert M, et al. Cancer Chemother Pharmacol. 2004;53:102-106; Dutreix C, Peng B, Mehring G, et al. Cancer Chemother Pharmacol. 2004;54:290-294; Smith PF, Bullock JM, Booker BM, et al. Pharmacother. 2004;24(11):1508-1514; Frye RF, Fitzgerald SM, La-gattuta TF, Hruska MW, Egorin MJ. Clin Pharmacol Thr. 2004;76:323-329).

Hence, in an alternative embodiment, the present invention provides a method of treating CML in a human patient comprising the steps of
(a) administering a predetermined fixed amount of Imatinib or a pharmaceutically acceptable salt thereof to the human CML patient in need thereof,
(b) collecting at least one blood sample from said patient within the first 12 months, especially the first 3 months, more especially the first 30 days of treatment,
(c) determining the Cmin value of N-{5-[4-(piperazino-methyl)-benzoylamido]-2-methylphenyl}-4-(3-pyridyl)-2-pyrimidine-amine (CGP74588), and
(d) adjusting the dose of Imatinib or a pharmaceutically acceptable salt thereof in a manner that a Cmin value of at least 150, especially between about 150 and about 800 ng/mL of N-{5-[4-(piperazino-methyl)-benzoylamido]-2-methylphenyl}-4-(3-pyridyl)-2-pyrimidine-amine is achieved in said patient.

In the latter method the dose of the pharmaceutically acceptable salt of Imatinib is adjusted in a manner that a Cmin between about 250 and about 700 ng/mL of N-{5-[4-(piperazino-methyl)-benzoylamido]-2-methylphenyl}-4-(3-pyridyl)-2-pyrimidine-amine is achieved in said patient.

Additional aspects covered by the present invention are as follows:
(1) A method of treating chronic myeloid leukemia (CML) in a human patient population comprising the steps of
   (a) administering a predetermined fixed therapeutically effective amount of an Bcr-Abl tyrosine kinase inhibitor or a pharmaceutically acceptable salt thereof to human CML patients in need thereof,
   (b) collecting at least one blood sample from said patients,
   (c) determining the plasma trough level (Cmin) of the Bcr-Abl tyrosine kinase inhibitor or of a metabolite thereof as well as the MMR rates,
   (d) assessing a discrimination potential of trough plasma concentrations for MMR and identifying a Cmin threshold for optimal sensitivity and specificity, e.g. by Receiver Operating Characteristic (ROC) curve analysis, and
   (e) adjusting the dose of the inhibitor of the Bcr-Abl tyrosine kinase or a pharmaceutically acceptable salt thereof applied to the individual patients from said patient population and, optionally, future CML patients in a manner that a Cmin is achieved in each single patient equal to or higher than the Cmin threshold obtained under step (d);
(2) a method of treating chronic myeloid leukemia (CML) in a human patient comprising the steps of
   (a) administering a therapeutically effective amount of a pharmaceutically acceptable salt of Imatinib to the human CML patient in need thereof,
   (b) collecting at least one blood sample from said patient within the first 12 months of treatment,
   (c) determining the plasma trough level (Cmin) of Imatinib, and
   (d) adjusting the dose of a pharmaceutically acceptable salt of Imatinib in a manner that a Cmin between about 800 and about 3500 ng/mL of Imatinib is achieved in said patient; and
(3) a method of treating chronic myeloid leukemia (CML) in a human patient comprising the steps of
   (a) administering a therapeutically effective amount of a pharmaceutically acceptable salt of Imatinib to the human CML patient in need thereof,
   (b) collecting at least one blood sample from said patient within the first 12 months of treatment,
   (c) determining the plasma trough level (Cmin) of N-{5-[4-(piperazino-methyl)-benzoylamido]-2-methylphenyl}-4-(3-pyridyl)-2-pyrimidine-amine, and
   (d) adjusting the dose of a pharmaceutically acceptable salt of Imatinib in a manner that a Cmin between about 150 and about 800 ng/mL of N-{5-[4-(piperazino-methyl)-benzoylamido]-2-methylphenyl}-4-(3-pyridyl)-2-pyrimidine-amine is achieved in said patient.

Furthermore, the present invention relates to the use of a Bcr-Abl tyrosine kinase inhibitor or a pharmaceutically acceptable salt thereof for the manufacture of a medicament for the treatment of a Ph+ leukemia, wherein
(a) a predetermined fixed amount of the Bcr-Abl tyrosine kinase inhibitor or a pharmaceutically acceptable salt thereof is administered to human patients suffering from a Ph+ leukemia,
(b) at least one blood sample is collected from said patients,
(c) the plasma trough level (Cmin) of the Bcr-Abl tyrosine kinase inhibitor or of a metabolite thereof as well as the MMR rates is determined,
(d) a discrimination potential of trough plasma concentrations for MMR and identifying a Cmin threshold for optimal sensitivity and specificity is assessed and
(e) the dose of the inhibitor of the Bcr-Abl tyrosine kinase or a pharmaceutically acceptable salt thereof applied to the individual patients from said patient population and, optionally, future patients suffering from a Ph+ leukemia is adjusted in a manner that a Cmin is achieved in each single patient equal to or higher than the Cmin threshold obtained under step (d).

The Ph+ leukemia is preferably CML orPh+ ALL. The at least one blood sample is collected within the first 12 months of treatment, especially within the first 3 months, in particular within the first 30 days of treatment.

Furthermore, the present invention relates to the use of Imatinib or a pharmaceutically acceptable salt thereof for the manufacture of a medicament for the treatment of a Ph+ leukemia, wherein
(a) a predetermined fixed amount of Imatinib or a pharmaceutically acceptable salt thereof, e.g. an oral daily dose 400 mg or 800 mg of the mono-mesylate salt of Imatinib, is administered to the human patient suffering from a Ph+ leukemia,
(b) at least one blood sample from said patient is collected within the first 12 months, especially within the first 3 months, e.g. within the first 30 days, of treatment,
(c) the plasma trough level (Cmin) of Imatinib is determined, and
(d) the dose of Imatinib or a pharmaceutically acceptable salt thereof is adjusted in a manner that a Cmin of at least about 800, especially between about 800 and about 3500, ng/mL of Imatinib, in particular between about 1000 and about 3000 ng/mL of Imatinib, is achieved in said patient. The Ph+ leukemia is Philadelphia chromosome Ph+ ALL or, preferably, CML.

In another aspect, the present invention relates to the use of Imatinib or a pharmaceutically acceptable salt thereof for the manufacture of a medicament for the treatment of a Ph+ leukemia, wherein
(a) a predetermined fixed amount of Imatinib or a pharmaceutically acceptable salt thereof, e.g. an oral daily dose 400 mg or 800 mg of the mono-mesylate salt of Imatinib, is administered to the human patient suffering from a Ph+ leukemia,
(b) at least one blood sample from said patient is collected within the first 12 months, especially within the first 3 months, e.g. within the first 30 days, of treatment,
(c) the plasma trough level (Cmin) of N-{5-[4-(piperazino-methyl)-benzoylamido]-2-methylphenyl}-4-(3-pyridyl)-2-pyrimidine-amine is determined, and
(d) the dose of Imatinib or a pharmaceutically acceptable salt thereof is adjusted in a manner that a Cmin of at least about 150, especially between about 150 and about 800, ng/mL, preferably between about 250 and about 700 ng/mL, of N-{5-[4-(piperazino-methyl)-benzoylamido]-2-methylphenyl}-4-(3-pyridyl)-2-pyrimidine-amine is achieved in said patient.

In one embodiment of the present invention, the predetermined fixed amount referred to herein under step (a) represents a therapeutically effective amount.

Throughout the present invention, preferably the mono-mesylate salt of Imatinib is used in step (a), e.g. in an oral daily dose of between about 200 and about 800 mg, preferably in a daily dose of about 400 mg.

The methods described herein are particularly beneficial for CML patients having an Intermediate Sokal score (ISS). Methods to determine the ISS are known to the person skilled in the art.

Another important aspect of the present invention is the use of Imatinib or a pharmaceutically acceptable salt thereof, especially Imatinib mesylate, for the manufacture of a medicament for the treatment of a Ph+ leukemia, wherein the dose of the pharmaceutically acceptable salt is adjusted in a manner that a Cmin of at least 800 ng/mL, e.g. about 1000 ng/mL, of Imatinib is maintained in said patient.

### Short discussion of the Figures

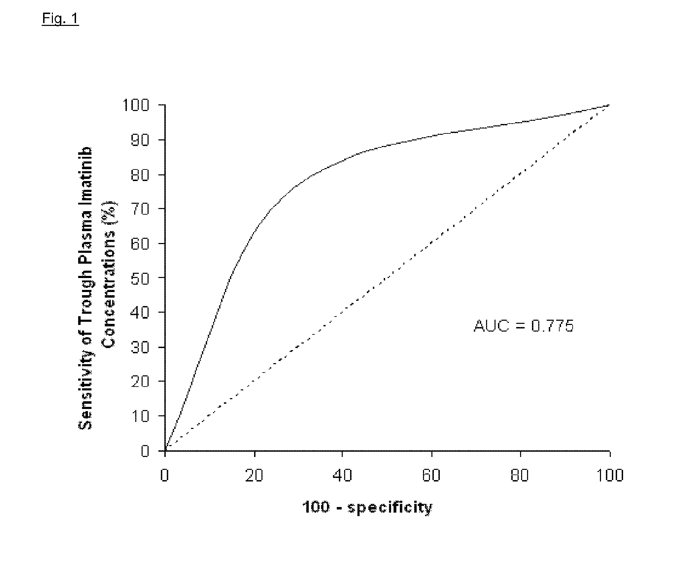

**Fig. 1****:** ROC curve analysis. Receiver operating characteristic (ROC) curve analysis was performed in order to assess a discrimination potential of trough plasma Imatinib concentrations for MMR, and to identify a plasma threshold for optimal sensitivity and specificity. The area under the ROC curve (AUC) was 0.775, with best sensitivity (76.5 percent) and specificity (70.6 percent) at a plasma threshold of 1002 ng per millilitre. This 1002 ng per millilitre threshold was significantly associated with the presence of MMR (adjusted odds ratio, 7.83; 95 percent confidence interval, 2.58 to 23.76; P<0.001).

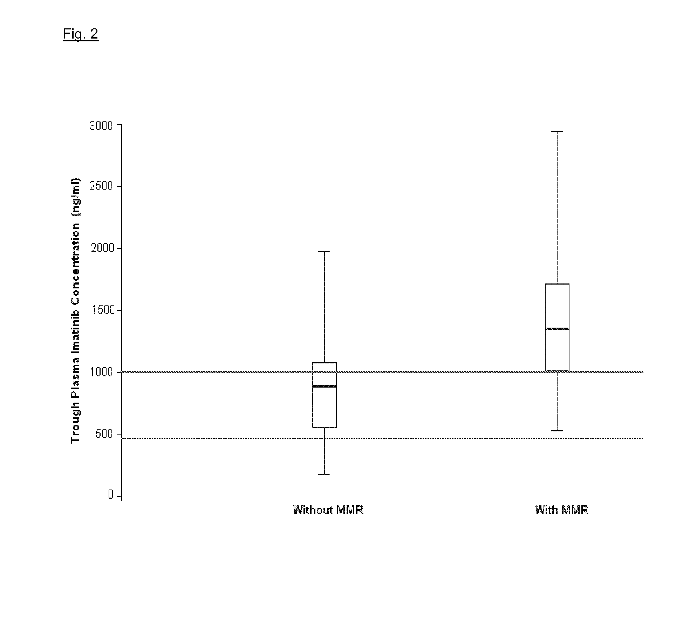

**Fig. 2****:** Box-plot graph. MMR means major molecular response (3 log reduction in *BCR-ABL* transcript levels). The graph shows the dispersion around the median, for patients with MMR (34 patients, median=1350.2 ng per millilitre) and those without (34 patients, median=885.5 ng per millilitre). The line across each box is the median. The bottom edge is the first quartile and the top edge is the third quartile. The error bars represent minimal and maximal values. The lower line shows the 493.6 ng per millilitre (1 micromol per liter) target concentration, required to result in *BCR-ABL-*positive cell death *in vitro.* The upper line shows the 1002 ng per millilitre efficient plasma threshold for trough Imatinib concentrations in CML treatment.

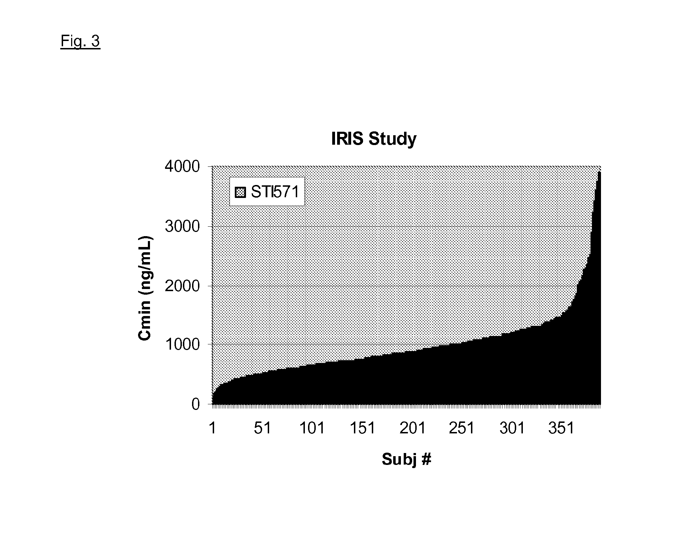

**Fig. 3** shows the variability of the Cmin level of Imatinib observed in the IRIS study in patients all obtaining the same daily dose of 400 mg Imatinib mesylate.
**Fig. 4** shows the distribution of imatinib trough levels at 400 mg daily on Day 29 (n=351).
**Fig. 5** shows the Imatinib trough level by body weight (BW) or body surface area (BSA).
**Fig. 6** shows CCR or MMR by imatinib trough level (Day 29).
**Fig. 7** shows plasma trough levels corresponding to achievement of CCR and non-CCR in CML-CP patients. Top and bottom walls of each box represent 75th and 25th percentiles. Whiskers (error bars) above and below the box indicate the 90th and 10th percentiles, and the dots represent 95th and 5th percentiles.
**Fig. 8** depicts event free survival (EFS) grouped based on Imatinib PK trough level quartiles.

Bcr-Abl tyrosine kinase inhibitor useful for the present invention are, e.g. compounds of formula I, 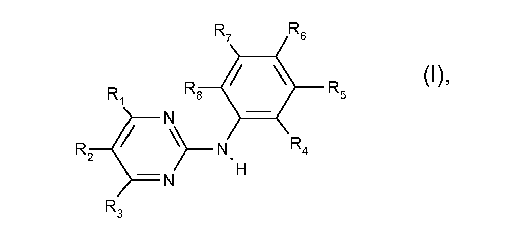 wherein
R₁ is 4-pyrazinyl; 1-methyl-1 H-pyrrolyl; amino- or amino-lower alkyl-substituted phenyl, wherein the amino group in each case is free, alkylated or acylated; 1 H-indolyl or 1 H-imidazolyl bonded at a five-membered ring carbon atom; or unsubstituted or lower alkylsubstituted pyridyl bonded at a ring carbon atom and unsubstituted or substituted at the nitrogen atom by oxygen;
R₂ and R₃ are each independently of the other hydrogen or lower alkyl;
one or two of the radicals R₄, R₅, R₆, R₇ and R₈ are each nitro, fluoro-substituted lower alkoxy or a radical of formula II

   -N(R₉)-C(=X)-(Y)ₙ-R₁₀ (II),

   wherein
   R₉ is hydrogen or lower alkyl,
   X is oxo, thio, imino, N-lower alkyl-imino, hydroximino or O-lower alkyl-hydroximino,
   Y is oxygen or the group NH,
   n is 0 or 1 and
   R₁₀ is an aliphatic radical having at least 5 carbon atoms, or an aromatic, aromatic-aliphatic, cycloaliphatic, cycloaliphatic-aliphatic, heterocyclic or heterocyclic-aliphatic radical,
      and the remaining radicals R₄, R₅, R₆, R₇ and R₈ are each independently of the others hydrogen, lower alkyl that is unsubstituted or substituted by free or alkylated amino, piperazinyl, piperidinyl, pyrrolidinyl or by morpholinyl, or lower alkanoyl, trifluoromethyl, free, etherified or esterifed hydroxy, free, alkylated or acylated amino or free or esterified carboxy,
      or of a salt of such a compound having at least one salt-forming group.

The compounds of formula I are generically and specifically disclosed in the patent applications US 5,521,184, in particular in the compound claims and the final products of the working examples, the subject-matter of which is hereby incorporated into the present application by reference. In the above definition of the compound of formula I the radicals and symbols have the meanings as provided in US 5,521,184. Preferably, the compound of formula I is 4-(4-methylpiperazin-1-ylmethyl)-N-[4-methyl-3-(4-(pyridin-3-yl)pyrimidin-2-ylamino)phenyl]-benzamide (Imatinib). Imatinib can also be prepared in accordance with the processes disclosed in WO03/066613.

For the purpose of the present invention, Imatinib is preferably applied in the form of its mono-mesylate salt. Imatinib mono-mesylate can also be prepared in accordance with the processes disclosed in US 6,894,051 the subject-matter of which is hereby incorporated into the present application by reference. Comprised are likewise the corresponding poly-morphs, e.g. crystal modifications, which are disclosed therein.

In step (a) of the method described above, in particular a daily dose of between about 200 and about 800 mg, e.g. 400 mg, of the mono-mesylate salt of Imatinib is administered orally. Imatinib mono-mesylate can be administered in dosage forms as described in US 5,521,184, US 6,894,051, US 2005-0267125 or WO2006/121941.

Further suitable Bcr-Abl tyrosine kinase inhibitor being useful for the present invention are disclosed in US 2006-0142577, WO2004/005281, WO2005/123719, WO2006/034833 and WO2000/62778. The latter patent application discloses Dasatanib (BMS 354825). In one embodiment of the present invention, Dasatanib is used as the Bcr-Abl tyrosine kinase inhibitor in the methods described herein.

The collecting of a blood sample from CML patients required under step (b) of the methods described herein can be accomplished by standard procedures being state of the art. A suitable procedure for the determination of the plasma trough level Cmin of Imatinib and N-{5-[4-(piperazino-methyl)-benzoylamido]-2-methylphenyl}-4-(3-pyridyl)-2-pyrimidine-amine was described by R. Bakhtiar R et al. in J Chromatogr B Analyt Technol Biomed Life Sci. 2002 Mar 5;768(2):325-40.

### Examples

The following examples are illustrative, but do not serve to limit the scope of the invention described herein. The examples are meant only to suggest a method of practicing the present invention. Quantities of ingredients, represented by percentage by weight of the pharmaceutical composition, used in each example are set forth below.

### EXAMPLE 1 - Study Design, Statistical Analysis and Results of Bordeaux Study

### PATIENTS

Patients included in the study were in chronic-phase or accelerated-phase CML. They were followed in the Department of Hematology and Blood Diseases of the Bordeaux Hospital Center (CHU de Bordeaux) and in the Institut Bergonié, Regional Cancer Center. All patients were treated orally with standard-dose Imatinib mesylate (i.e. 400 mg or 600 mg once daily for patients with chronic-phase or accelerated-phase CML, respectively) for at least 12 months. In the study population, blood sample collections were carried out between June 2004 and March 2006, in order to test the association between trough plasma Imatinib concentrations and response to treatment. Exclusion criteria were initiation of Imatinib mesylate therapy less than one year before, blast crisis before or during Imatinib mesylate therapy, blood collection performed out of the trough concentration time limits, poor compliance to treatment, identification of gene mutation(s) in the kinase domain of Bcr-Abl.

### QUANTIFICATION OF RESPONSE TO THERAPY

The cytogenetic response to Imatinib mesylate therapy was assessed using a conventional cytogenetic analysis of bone marrow metaphases. Cytogenetic responders were defined as having CCR, i.e. 0 percent of Philadelphia-chromosome-positive metaphases among at least 25 cells in metaphase in the bone marrow aspirate (Colombat M, Fort MP, Chollet C, et al. Haematologica 2006;91:162-8.). Real-time quantitative reverse-transcriptase polymerase chain reaction assay was used to assess *BCR-ABL* transcript levels and quantify the molecular response (Colombat M, Fort MP, Chollet C, et al. see above). Briefly, EDTA-anticoagulated peripheral blood was collected to perform RNA extraction followed by real-time quantitative reverse-transcriptase polymerase chain reaction. Total RNA was extracted from peripheral blood cells of patients using standard methods. Quantification of *BCR-ABL* transcripts was performed according to recommendations recently proposed for harmonization of results (Hughes TP, Deininger M, Hochhaus A, et al. Blood 2006;108:28-37.). Hence, the results were given using *ABL* gene as the gene control and were expressed as percentage of *BCR-ABL* / *ABL.* A standardized baseline was calculated by measuring the ratio of *BCR-ABL* / *ABL* in 40 chronic-phase CML patients from blood collected before any treatment. For each sample, this baseline was used to evaluate and assess the *BCR-ABL* transcript reduction. A MMR was defined as a reduction in *BCR-ABL* transcript levels of at least 3 log after 12 months of Imatinib mesylate therapy (Hughes TP, Kaeda J, Branford S, et al. International Randomised Study of Interferon versus STI571 (IRIS) Study Group. N Engl J Med 2003;349:1423-32.).

### QUANTIFICATION OF TROUGH PLASMA IMATINIB CONCENTRATIONS

Blood samples for Imatinib plasma quantification were collected at steady-state between 21 and 27 hours after last drug administration. Trough plasma Imatinib concentrations were determined using high-performance liquid chromatography coupled to electrospray-ionisation tandem mass spectrometry (Titier K, Picard S, Ducint D, et al. Ther Drug Monit 2005;27:634-40. [Erratum, Ther Drug Monit 2005;27:810.]). Pure reference samples of Imatinib mesylate and its internal standard (Imatinib-D8) were kindly donated by Novartis (Rueil-Malmaison, France). The sample preparation consisted of a liquid-liquid extraction, performed from 200 microliters of plasma. Then, 5 microliters of extract were injected onto the chromatographic system. The high-performance liquid chromatography unit consisted of an Alliance® 2690 separation module (Waters, Milford, MA, USA) piloted by the Masslynx® software. Imatinib and Imatinib-D8 were separated on a reversed-phase column (X-Terra® RP18, [100x2.1 millimeters, 5 micrometers], Waters) with a gradient of acetonitrile-formiate buffer. Total run time analysis was 6 minutes at a flow rate of 0.3 millilitre per minute. Imatinib quantification was performed using tandem mass spectrometry (QuattroMicro®, Watters, Milford MA, USA) with an electrospray-ionisation interface in positive ion mode. The cone voltage was set at 40 volts for Imatinib and its internal standard, and the collision energy was set at 30 electron volts for the two compounds. Imatinib and Imatinib-D8 were detected in multiple reaction monitoring transitions. To quantify Imatinib, the peak area corresponding to the m/z 494.2 → 394.1 reaction (Imatinib) was measured relative to that of the m/z 502.2 → 394.1 reaction (internal standard). Imatinib identification was confirmed by a second specific multiple reaction monitoring transition : *m*/*z* 494.2 → 217.2.

### STATISTICAL ANALYSIS

Regarding quantitative variables, comparisons of means between two groups were performed using Student's *t* test or the Wilcoxon rank test when appropriate. In the presence of more than two groups, an analysis of variance or a Kruskal-Wallis test was used. Regarding qualitative variables, comparisons of proportions were performed using a χ² test or exact Fisher test when appropriate. At steady-state, variability in trough plasma Imatinib concentrations was expressed by the following parameters : mean trough plasma concentrations, standard deviation (SD), coefficient of variation, median, first and third quartiles, maximum and minimum measured trough plasma concentrations.
For CCR and MMR successively, group of responders and group of patients that did not respond were compared with regard to their mean trough plasma Imatinib concentrations. A possible association was investigated between MMR and the following variables : quantitative features such as age and Sokal score ; and qualitative features such as sex, Sokal risk group, accelerated-phase CML at initiation of Imatinib mesylate therapy, administration of interferon before Imatinib mesylate treatment, daily dose level of Imatinib mesylate. Any relationship between *BCR-ABL* transcript levels and the range time from date of Imatinib mesylate treatment initiation to date of molecular analysis was evaluated.
ROC curve analysis was performed with a multivariate logistic regression model, adjusted on age and sex, in order to assess a discrimination potential of trough plasma Imatinib concentrations for MMR, and to identify a plasma threshold for optimal sensitivity and specificity. Results were expressed as adjusted odds ratio; 95 percent confidence interval; P value of Wald test.
Two-sided P values were reported for statistical tests (P<0.05 indicated significance). All analyses were done using SAS Software (version 9.1, Cary, NC, USA).

### PATIENTS INCLUDED FOR INVESTIGATION

Ninety-five CML patients were considered for participation in the study. One patient was excluded because he was found in blast crisis. Twenty-four patients were excluded because of an inadequate blood collection i.e. performed out of time limits for determination of trough imatinib concentration. One patient was excluded because of recognized poor compliance to imatinib therapy : this patient failed to respond haematologically and had plasma levels of imatinib below 10 ng per millilitre. One patient was excluded because a G250E mutation was identified in the kinase domain of Bcr-Abl. Finally, 68 CML patients were included for investigation. Fifty patients and 18 patients were respectively treated with 400 mg and 600 mg imatinib once daily.

### VARIABILITY IN TROUGH PLASMA IMATINIB CONCENTRATIONS AMONG PATIENTS

Variations in trough plasma Imatinib concentrations for each daily dose regimen of Imatinib mesylate (400 mg and 600 mg) are shown in Table 1.1. These concentrations of Imatinib were highly variable ranging from 181 to 2947 ng per millilitre, confirming the high variability previously described between subjects in trough plasma Imatinib concentrations for a given daily dose (Peng B, Hayes M, Resta D, et al. J Clin Oncol 2004;22:935-42.)

**Table 1.1 Variability in trough plasma Imatinib concentrations among patients (n=68)*.**

| | **Dispersion around the mean** | | | **Median** | **Q 25** 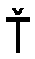 | **Q 75 ‡** | **Minimum** | **Maximum** |
|---|---|---|---|---|---|---|---|---|
| **Daily dose level** | Mean value | SD § | CV \| | | | | | |
| 400 mg | 1058 | 557 | 52.6 | 997 | 722 | 1285 | 181 | 2947 |
| 600 mg | 1444 | 710 | 49.2 | 1315 | 902 | 1629 | 603 | 2922 |

| | | | | | | | | |
|---|---|---|---|---|---|---|---|---|
| * Parameters of variations in trough plasma Imatinib concentrations are expressed in ng per millilitre and reported for each daily dose regimen of Imatinib mesylate: 400 mg (50 patients) and 600 mg (18 patients). These data result from the analysis of blood sample collections (using high-performance liquid chromatography coupled to electrospray-ionisation tandem mass spectrometry) performed, at steady-state, in the 68 CML patients included in the study. 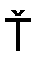 Q 25 is the first quartile. ‡ Q 75 is the third quartile. § SD is the standard deviation. \| CV is the coefficient of variation expressed in percent. | | | | | | | | |

### PATIENTS' CHARACTERISTICS ACCORDING TO RESPONSES TO IMATINIB

Of the 68 CML patients included for investigation, 56 achieved a CCR after at least one year's treatment. Mean (±SD) trough plasma Imatinib concentrations were 1123.3±616.6 ng per millilitre and 694.2±556.0 ng per millilitre in patients with CCR (56 patients) and without CCR (12 patients), respectively (P=0.02). Concerning the molecular response, main characteristics of the 68 CML patients, classified as those with or without MMR, are summarized in Table 1.2. Mean trough plasma Imatinib concentrations were significantly higher in the group with MMR (1452.1±649.1 ng per millilitre) than in the group without (869.3±427.5 ng per millilitre, P<0.001). No significant difference was found in the Imatinib mesylate daily dose between patients with or without MMR. Moreover, MMR to Imatinib was not related to the following features : clinical data (age, sex), accelerated-phase CML at initiation of Imatinib mesylate therapy, administration of interferon before Imatinib mesylate treatment. Furthermore, MMR to treatment was not statistically associated with the time elapsed between initiation of Imatinib mesylate therapy and molecular analysis : mean (±SD) values were 986.5±427 days and 966.5±560 days in groups with and without MMR, respectively (P=0.87). Four groups of patients were compared according to the date of their molecular analysis (using a Kruskal-Wallis test) : within 560 days of initiation of Imatinib mesylate treatment (16 patients), between 560 and 900 days after initiation of Imatinib mesylate treatment (18 patients), between 900 and 1325 days after (17 patients), more than 1325 days after initiation of Imatinib mesylate treatment (17 patients). The test showed that there was no significant difference in *BCR-ABL* transcript levels between the four groups (P=0.48). The rate of molecular response was therefore not time-dependent in our investigation.

**Table 1.2. Patients' characteristics according to molecular response to Imatinib therapy.**

| | **Without MMR *** | | **With MMR** | | **P value** 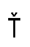 |
|---|---|---|---|---|---|
| **Characteristics** | No. ‡ | data § | No. | data | |
| **Quantitative features** | | | | | |
| | | | | | |
| Trough plasma imatinib concentrations \| | 34 | 869.3 ± 427.5 | 34 | 1452.1 ± 649.1 | < 0.001 |
| | | | | | |
| Age | 34 | 50.7 ± 13.6 | 34 | 51.7 ± 13.7 | 0.76 |
| | | | | | |
| Sokal score | 32 | 0.9 ± 0.4 | 33 | 1.0 ± 0.4 | 0.33 |
| | | | | | |
| | | | | | |
| **Qualitative features** | | | | | |
| | | | | | |
| Sex | | | | | 0.09 |
| Male gender | 24 | 70.6 | 17 | 50.0 | |
| Female gender | 10 | 29.4 | 17 | 50.0 | |
| | | | | | |
| Sokal risk group | | | | | 0.69 |
| < 0.8 | 15 | 44.1 | 14 | 41.2 | |
| [0.8-1.2] | 12 | 35.3 | 11 | 32.4 | |
| > 1.2 | 5 | 14.7 | 8 | 23.5 | |
| | | | | | |
| Accelerated-phase CML | | | | | 0.58 |
| no | 26 | 76.5 | 24 | 70.6 | |
| yes | 8 | 23.5 | 10 | 29.4 | |
| | | | | | |
| Interferon before imatinib | | | | | 0.62 |
| no | 15 | 44.1 | 13 | 38.2 | |
| yes | 19 | 55.9 | 21 | 61.8 | |
| | | | | | |
| Daily imatinib dose | | | | | 1.00 |
| 400 mg | 25 | 73.5 | 25 | 73.5 | |
| 600 mg | 9 | 26.5 | 9 | 26.5 | |

| | | | | | |
|---|---|---|---|---|---|
| * MMR means major molecular response (3 log reduction in BCR-ABL transcript levels). The main characteristics of the 68 CML patients are classified into those with or without MMR. 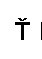 P value was assessed using Student's *t* test for quantitative variables and the χ² test for qualitative variables. ‡ No. is the number of patients. § Data are mean values (± standard deviation) for quantitative features. Data are proportions in percent for qualitative features. ¦ Trough plasma Imatinib concentrations are expressed in ng per millilitre. | | | | | |

### TROUGH PLASMA IMATINIB TRESHOLD FOR MAJOR MOLECULAR RESPONSE

The concentration-effect ROC curve analysis tested the discrimination potential of trough plasma Imatinib concentrations for MMR (Fig. 1). For the latter, the area under the ROC curve was 0.775, with best sensitivity (76.5 percent) and specificity (70.6 percent) at a plasma threshold of 1002 ng of Imatinib per millilitre. This 1002 ng per millilitre threshold was significantly associated with the presence of MMR (adjusted odds ratio, 7.83; 95 percent confidence interval, 2.58 to 23.76; P<0.001). Box-plots of trough plasma Imatinib concentrations showed the dispersion around the median (Fig. 2) for patients with MMR (34 patients, median=1350.2 ng per millilitre) and those without (34 patients, median=885.5 ng per millilitre). In the group with MMR, 26 patients (76.5 percent) of the 34 patients had trough plasma Imatinib concentrations exceeding the 1002 ng per millilitre threshold and there was no patient having trough plasma Imatinib concentrations below 493.6 ng per millilitre (1 micromol per liter) which is the initially described target concentration required to result in *BCR-ABL*-positive cell death *in vitro.* In the group without MMR, 24 patients (70.6 percent) of the 34 patients had trough plasma Imatinib concentrations below the 1002 ng per millilitre threshold and there were 7 patients (20.6 percent) having trough plasma Imatinib concentrations below 493.6 ng per millilitre (1 micromol per liter).

### EXAMPLE 2 - Analysis of IRIS Study Data

In this study, plasma trough levels of Imatinib at steady state following the first month of treatment (Day 29) proved to be a significant prognostic covariate for long term clinical responses in CML patients.

The variability of Imatinib exposure has clinical implications. Achievement of a CCR is a validated surrogate for clinical benefit in CML and an appropriate measure of initial antileukemic efficacy. The times to CCR and to MMR within CCR patients were significantly different between patients with different Imatinib plasma exposures grouped in quartiles (p<0.025). Patients achieving CCR by one year had steady state concentrations of Imatinib after one month that were higher than those not achieving CCR. Overall Cₘᵢₙ values were statistically significantly higher in patients who achieved CCR during the study (mean 1009 ng/mL vs 812 ng/mL). Thus, maintaining an Imatinib trough level at or above 1000 ng/mL might be important for CCR. This result is consistent with the threshold value of about 100 ng/mL found in Example 1.

In this analysis, we assumed patients maintained adherence to Imatinib therapy, during both the first and subsequent months. However, this is an unknown variable although patients submitted dosing records during this trial. While adherence to therapy is a critical determinant to accuracy and validity of pharmacokinetic analysis, it was reasonable to assume that the plasma levels of imatinib and CGP74588 were at steady state on Day 29 in this well monitored clinical study. Patients enrolled in this study were newly diagnosed with a life-threatening disease and in the early stages of treatment - a point when there was great incentive to take daily doses of Imatinib, a relatively well tolerated drug. High levels of nonadherence to Imatinib in this study were unlikely for most patients. Out of the 553 patients enrolled in the Imatinib treatment arm, nearly 20% of the patients had a dose escalation for a clinical reason to 600-800 mg daily doses, the median time to dose escalation was 22 months (Data not shown). Nonadherence to Imatinib has been documented in CML patients and could have impacted clinical responses and the correlation between clinical response and PK trough exposure.

We found a correlation between MMR rates and Imatinib exposure. The estimated MMR rate was significantly lower in patients with low Imatinib levels; only an estimated 25% of all patients with Imatinib levels <647 ng/mL achieved an MMR at 1 year, whereas 40% of patients with higher Imatinib levels achieved that response within 1 year. By 4 years, an estimated 53% of patients in Q1 had achieved MMR despite low steady state (day 29) Imatinib levels compared to 80% of patients in Q4 (and 72 % of patients within the inter-quartile range, IQ). MMR is prognostic for long term efficacy and survival. Patients who lack MMR have increased rates of disease progression. Thus early dose adjustment for patients with low Imatinib steady state levels may improve long term efficacy.

In addition to CCR and MMR response rates, imatinib PK trough level appeared to be somewhat correlated with event free survival (EFS) although no statistically significant difference was achieved. The event free survival is a complicated event which might be confound with many different factors such as accessibility of other treatments, intra-patient dose escalation in the later time of treatment period, etc. Nonetheless, patients with a low Imatinib trough level tend to have a poor EFS than patients with higher Imatinib levels. As expected, Imatinib exposure was correlated with the rate of discontinuation. Patients in the lower quartile had the highest discontinuation rate than inter and upper quartiles. Interestingly, one of the reasons for discontinuation was related to unsatisfactory therapeutic effect, which was consistent with the findings from the correlation analysis between clinical response (CCR or MMR) and imatinib quartile concentrations.

Our report describes Imatinib trough level at steady state. Similar results were observed for the major active metabolite, CGP74588. However, considering the relatively small contribution of the metabolite to Imatinib exposure (<20%), measurement of parent drug in the plasma represents the major active component for biological activity. If the metabolism of Imatinib were altered, for example by a CYP inducer or inhibitor, measurement of both Imatinib and metabolite might be necessary.

In conclusion, Imatinib steady-state plasma exposure measured following the first month of treatment with a standard 400 mg dose correlated with long term cytogenetic and molecular responses. Patient demographics including age, gender, and body size have minimal impact on Imatinib plasma exposure considering the large inter-patient variability of the exposure. Maintaining plasma trough levels at or above the mean population concentration of approximately 1000 ng/mL may be important for the CCR and MMR response, free survival, and satisfactory therapeutic efficacy in chronic phase CML patients. Any factors which might affect Imatinib exposure, such as drug absorption, metabolism, and interactions between prescribed medications, may thereby impact the ability to achieve a maximal therapeutic benefit. Information regarding Imatinib blood exposure during therapy has the potential to serve as a valuable tool and merits prospective validation.

### Methods

Patients included in this analysis were enrolled in the IRIS trial and randomly assigned to initial treatment with Imatinib at 400 mg/day. The study design and patient characteristics for all 553 patients randomized to Imatinib including age, gender, body weight, body surface area as well as outcomes have been described previously (O'Brien SG, Guilhot F, Larson RA, et al. N Eng J Med. 2003;348:994-1004.).

Rates of CCR (defined as 0% Ph+ metaphase cells out of at least 20 examined) in the study population and major molecular response (MMR, defined as ≥ 3 log reduction in BCR-ABL/BCR ratio from a standardized baseline) in subjects who achieved CCR before were reported previously (O'Brien SG, Guilhot F, Larson RA, et al., see above).

This Example focuses on those 351 patients with available PK measurements. Event-free survival (EFS) was evaluated up to 5 years and was measured from enrollment onto the clinical trial until any of the following events: death from any cause, loss of a MMR, loss of a complete hematologic response, or progression to accelerated or blast phase. Alive patients were censored for survival at last follow up. CCR were evaluated up to 5 years. Achievement of MMR was only analyzed up to 24 months after treatment start due to limited data after that point. The disposition of patients (with available PK information) and reasons for discontinuation after 5 year treatment were tabulated with respect to cross-over to other treatment arm, adverse events, unsatisfactory therapeutic effect, and other reasons (including abnormal procedure, no longer requires study drug (BMT), protocol violation, subject withdrew consent, lost to follow-up, and death).

### Pharmacokinetic Sample Analysis

Blood samples were collected prior to Imatinib dosing on Day 2 (i.e. 24 hours after the first dose) and again on Day 29 (steady state trough level). The plasma concentrations of Imatinib and CGP74588 were determined by liquid chromatography and tandem mass spectronomy (LC/MS/MS). The limit of quantification was 5 ng/ml for both Imatinib and CGP74588; the assay was fully validated (Bakhtiar R, Lohne J, Ramos L, Khemani L, Hayes M, Tse F; J Chromatog B Anal Technol Biomed Life Sci. 2002;768:325-40.) The accuracy and precision were 104% ± 6% at the lower limit of quantification and 99% ± 5% to 108% ± 5% over the entire concentration range of 4-10,000 ng/ml.

### Data Analysis

Trough plasma concentrations (Cₘᵢₙ value) of Imatinib and its metabolite after the first dose and at steady state were analyzed and correlation analysis was performed retrospectively with clinical responses including CCR and MMR, as well as patient disposition after 2 and 5 years of treatment. Correlation of PK trough levels with age, gender, body weight and body surface area was assessed. Plasma trough levels of both Imatinib and CGP74588 on Days 2 and 29 were grouped into four quartiles. The lower quartile (Q1) includes data on the 25% of patients with the lowest observed concentration values, whereas quartiles Q2 and Q3 extend 25% below and above the median concentration, respectively. The upper quartile (Q4) includes the 25% of patients with the highest concentration values. The central 50% of the data, i.e. excluding Q1 and Q4, were combined for all analyses and are jointly referred to as intermediate quartiles (IQ). These three groups (Q1, IQ and Q4) were used for stratification as appropriate. The cytogenetic and molecular response rates were estimated using the Kaplan-Meier method, and strata exploratively compared by the log-rank test. The correlation between trough levels and demographic variables was evaluated by means of Spearman's rank correlation coefficient.

### Results

### Demographics and plasma trough levels of imatinib and its metabolite

Pharmacokinetic data were available from a total of 351 patients (221 males and 130 females). The mean body weight was 85.9 ± 16.8 (SD) kg for males (median, 83.6, and range, 52.9 to 163.3) and 72.4 ± 18.1 kg for females (median, 68.9, and range, 40.0 to 133.0). The body surface area (BSA) was 2.0 ± 0.2 m² for males (median, 2.0 and range, 1.53 to 2.8) and 1.8 ± 0.2 m² for females (median, 1.75 and range, 1.35 to 2.54). The median age of the population was 50 years (range, 18 to 70 years) Of the 351 patients who had evaluable samples in the PK sub-study, 238 remain on study (67.8%), 10 crossed over (2.8%), 113 (32.2%) discontinued Imatinib on study because of unsatisfactory therapeutic effect (n=51,14.5%), adverse events (n=15, 4.3%), death (n=6, 1.7%), bone marrow transplantation (n=11, 3.1 %), withdrawal of consent (n=15, 4.3), or other reasons such as abnormal procedures, protocol violation, lost to follow up, or administrative problems (n=15, 4.3%).

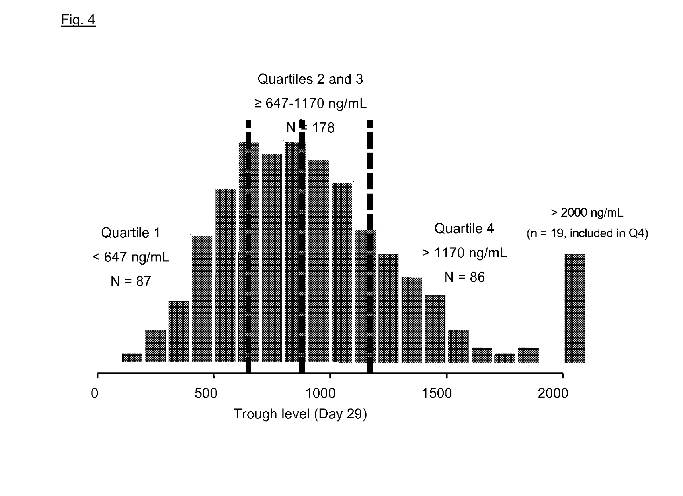

Following the 1^{st} first 400 mg dose, the 24-hour trough concentrations of Imatinib and CGP74588 were 517.7 ± 369.6 ng/mL and 82.7 ± 47.4 ng/mL, respectively. On day 29, the trough concentrations of Imatinib and CGP74588 were 979.0 ± 529.6 ng/mL and 241.9 ± 105.5 ng/mL, respectively; the metabolite to parent drug concentration ratio was 0.268 ± 0.085 (n=351). Based on the trough levels on days 2 and 29 on the same subject, the accumulation ratio to steady state was estimated to be 2.21 ± 1.15 for Imatinib and 3.38 ± 1.54 for CGP74588. The distribution of the trough concentrations of Imatinib at steady state is shown in Figure 4. There were 19 patients with Day 29 trough levels >2000 ng/ml included in the 4^{th} quartile for analysis.

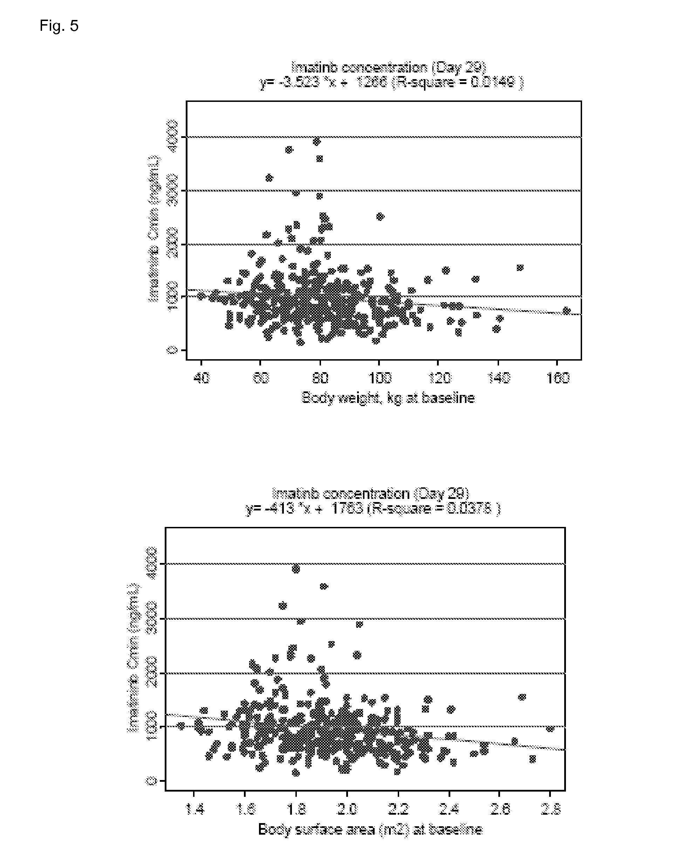

The plasma trough level of Imatinib was slightly higher in females than males (1078±514.5 ng/mL vs 921±530.8 ng/mL, respectively, and differed by 17.2%), probably due to body weight differences (18.7%) between genders. Plasma trough levels of the metabolite CGP74588 followed a similar pattern, while the metabolite/parent drug ratio was the same in males and females. There was a weak correlation between steady-state trough levels of Imatinib and both body weight (r² = 0.015) and BSA (r² = 0.038) as shown in Figure 5. Assuming a simple linear relationship between body weight and trough level, an increase from 40 kg to 120 kg in weight would result in an estimated decrease in trough level of approximately 280 ng/ml. There was also a weak correlation between trough levels (or metabolite/parent drug ratio) and the age of patients (r² = 0.02). Again making a simplifying assumption of linear relationship, trough levels of Imatinib increased by 295 ng/ml as age increased from 20 years to 70 years old. However, due to the large variability in plasma trough level between individuals, these effects of age, gender, and BW or BSA on Imatinib trough exposure are not likely to be clinically significant.

### Correlation of PK exposure with clinical responses

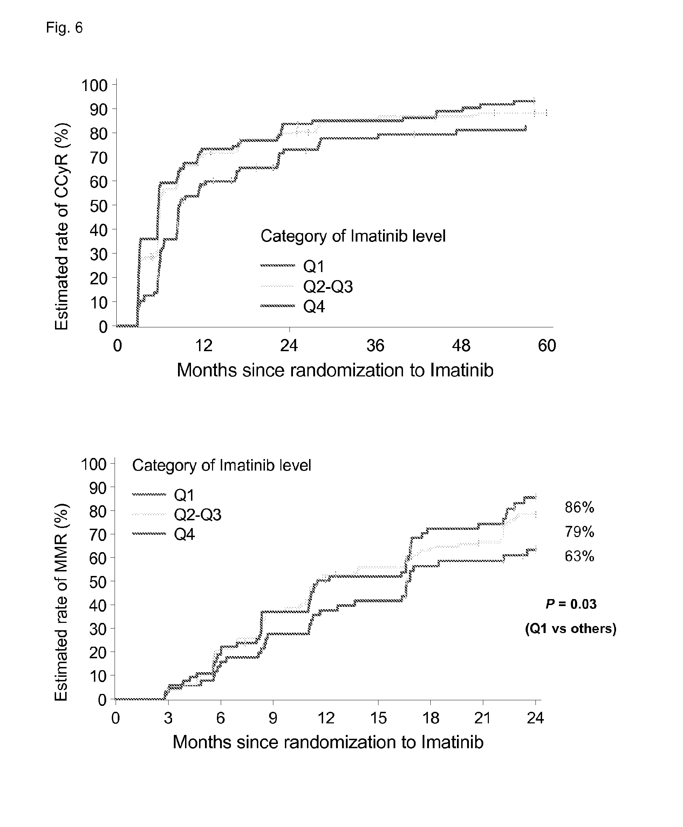

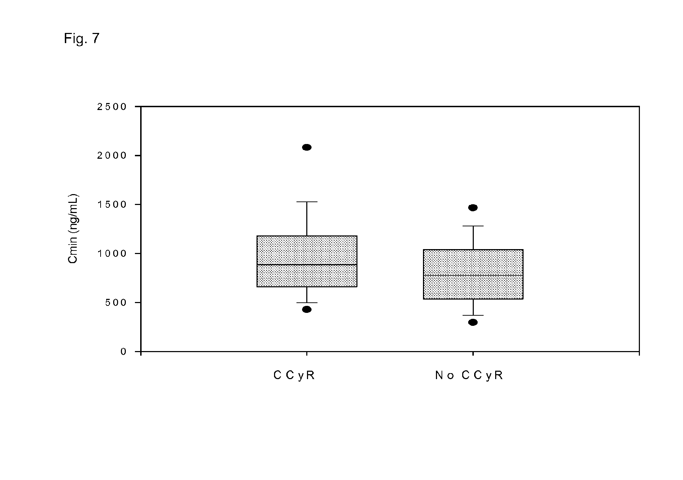

Table 2.1 lists the steady state trough levels of Imatinib, CGP74588, and their ratio grouped by quartiles. The trough exposures in Q2 and Q3 were combined as IQ to represent the central 50% of the population. Figure 6 (upper panel) shows that CCR response rates at 5 years were significantly different among different Imatinib trough level quartiles (p=0.0125). The difference was attributed mainly to a lower CCR rate in the Q1 group (p=0.005, Q1 vs others). A similar trend was observed for MMR rates at 2 years with respect to steady state plasma exposure levels. Patients in Q1 had a lower MMR rate than other groups combined, although not statistically significant difference was achieved among the three individual quartile groups (p = 0.08). The imatinib trough exposure in patients who eventually achieved CCR was significantly higher than those who did not achieve CCR, 1009±544 ng/mL vs 812±409 ng/mL, respectively (p=0.01 Figure 7). No significant difference in PK exposure was observed between those MMR responders and non-MMR responders. Statistical analysis of MMR rates was performed up to 24 months due to limited data thereafter.

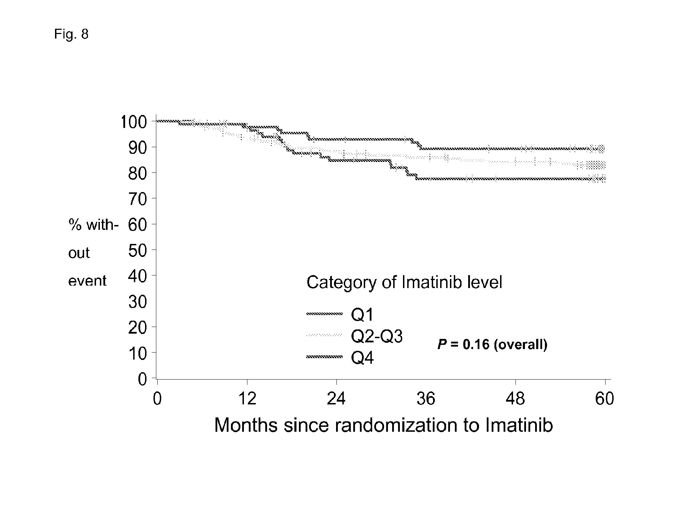

There appeared to be a trend in event free survival (EFS) with respect to Imatinib trough levels, a relatively poorer EFS in Q1 group than other quartiles. However, no statistically significant differences were achieved with the available dataset (Figure 8). A similar trend of correlation between patients disposition (or discontinuation) and PK trough levels was observed (Table 2.3). After 2 years treatment, the number of patients ongoing was low in Q1 group, 75.9%, comparing with 84.3% and 89.5% in IQ and Q4 groups. After 5 year treatment, the number of patients ongoing was 58.6%, 72.5%, and 76.7%, for Q1, IQ, and Q4, respectively. The main reason for discontinuation seems to be related to unsatisfactory therapeutic effect, 10.3%, 6.2%, and 4.7% in Q1, IQ, and Q4, respectively, after 2 year treatment, and 18.4%, 14.6%, and 8.1%, respectively after 5 year treatment. The adverse events- or death-related discontinuation rate was similar between different quartile groups after either 2 or 4 years treatment. There were no patients in Q4 group crossed over to other treatment arm (Interferon arm) as compared with 4.6% and 3.4% in the Q1 and IQ groups, respectively. Cross-over occurred mostly within the first one or two years after starting the treatment.

The clinical response (CCR, MMR or survival) or patient disposition was also correlated with the trough levels of metabolite CGP74588, since the parent drug and metabolite levels were highly correlated (0.76, Spearman correlation coefficient). The trough plasma level following the 1^{st} dose also showed a correlation with CCR and MMR responses, but appeared to be less predictive than the trough level at steady state.

**Table 2.1 Steady-state trough levels (Mean (±SD) [range]) of Imatinib and CGP74588 by quartiles**

| **Day 29 Data** | **Overall N=351** | **Quartile 1 N=87** | **Quartiles 2 and 3 N=178** | **Quartile 4 N=86** |
|---|---|---|---|---|
| Imatinib (ng/mL) | 979 (±529.6) | 490 (±119.7) | 889 (±148.2) | 1661 (±602.0) |
| | [153, 3910] | [153, 644] | [647, 1170] | [1180, 3910] |
| CGP74588 (ng/mL) | 242 (±105.5) | 153 (±48.5) | 236 (±65.8) | 343 (±126.1) |
| | [50.2, 841] | [50.2, 322] | [105, 455] | [160, 841] |
| CGP74588/Imatinib | 0.27(±0.085) | 0.32 (±0.106) | 0.27 (±0.068) | 0.21 (±0.052) |
| | [0.11, 0.84] | [0.15, 0.84] | [0.11,0.51] | [0.13, 0.36] |

**Table 2.2 CCR and MMR rates (%) at different steady-state Imatinib trough level quartiles**

| **Outcomes** | **Quartile 1 (n=87)** | **Quartiles 2 and 3 (n=178)** | **Quartile 4 (n=86)** |
|---|---|---|---|
| CCR (%[95% CI]) | | | |
| 1 year | 59 [48, 70]* | 71 [64, 78] | 73 [63, 83] |
| 2 years | 73 [63, 83] | 80 [73, 86] | 84 [75, 92] |
| 4 years | 81 [71, 91] | 87 [81, 93] | 89 [82, 96] |
| MMR (%) in pts with CCR | | | |
| 1 year | 43 [28, 59] | 56 [47, 66] | 55 [41, 68] |
| 2 years | 63 [49, 78] | 78 [69, 86] | 86 [76, 96] |
| 4 years | 65 [50, 80] | 83 [75, 91] | 90 [81, 100] |
| Time to CCR | 8.3 [2.7, 56.9] | 5.7 [2.7, 50.4] | 5.6 [2.8, 55.3] |
| (Month [95% Cl]) | | | |
| Time to MMR in pts with CCR (months) | 16.7 [2.8, 59.0] | 11.5 [2.7, 59.0] | 12.2 [2.8, 52.4] |

**Table 2.3 Patients disposition after 2 and 5 year treatment grouped based on steady-state Imatinib trough level**

| **Outcomes** | **Quartile 1 (n=87) n (%)** | **Quartiles 2 and 3 (n=178) n (%)** | **Quartile 4 (n=86) n (%)** |
|---|---|---|---|
| **Disposition after 2 years** | | | |
| Number of patients ongoing | 66 (75.9) | 150 (84.3) | 77 (89.5) |
| Crossed-over to other treatment arm | 4 (4.6) | 6 (3.4) | 0 (0) |
| Discontinued treatment | | | |
| 1. Unsatisfactory effect | 9 (10.3) | 11 (6.2) | 4 (4.7) |
| 2. Adverse event(s) | 3 (3.4) | 5 (2.8) | 2 (2.3) |
| 3. Death | 0 | 3(1.7) | 1 (1.2) |
| 4. Others* | 9 (10.3) | 9 (5.1) | 2 (2.3) |
| **Disposition after 5 years** | | | |
| Number of patients ongoing | 51 (58.6) | 129 (72.5) | 66 (76.7) |
| Crossed-over to other treatment arm | 4 (4.6) | 6 (3.4) | 0 (0) |
| Discontinued treatment | | | |
| 1. Unsatisfactory effect | 16(18.4) | 26(14.6) | 7(8.1) |
| 2. Adverse event(s) | 4 (4.6) | 5(2.8) | 6 (7.0) |
| 3. Death | 1 (1.1) | 4(2.2) | 1 (1.2) |
| 4. Others* | 15 (17.2) | 14 (7.9) | 6 (7.0) |

| | | | |
|---|---|---|---|
| *Others include: abnormal procedure, no longer requires study drug (BMT), protocol violation, subject withdrew consent, loss to follow-up, administrative problems. | | | |

## Claims

1. Use of Bcr-Abl tyrosine kinase inhibitor or a pharmaceutically acceptable salt thereof for the manufacture of a medicament for the treatment of a Ph+ leukemia, wherein
(a) a predetermined fixed amount of the Bcr-Abl tyrosine kinase inhibitor or a pharmaceutically acceptable salt thereof is administered to human patients suffering from a Ph+ leukemia,
(b) at least one blood sample is collected from said patients,
(c) the plasma trough level (Cmin) of the Bcr-Abl tyrosine kinase inhibitor or of a metabolite thereof as well as the MMR rates is determined,
(d) a discrimination potential of trough plasma concentrations for MMR and identifying a Cmin threshold for optimal sensitivity and specificity is assessed and
(e) the dose of the inhibitor of the Bcr-Abl tyrosine kinase or a pharmaceutically acceptable salt thereof applied to the individual patients from said patient population and, optionally, future patients suffering from a Ph+ leukemia is adjusted in a manner that a Cmin is achieved in each single patient equal to or higher than the Cmin threshold obtained under step (d).

2. The use according to claim 1 wherein the Ph+ leukemia is chronic myeloid leukemia (CML).

3. The use according to claim 1 wherein the Ph+ leukemia is Philadelphia chromosome positive acute lymphoblastic leukemia (Ph+ ALL).

4. The use according to claim 2 or 3 wherein the at least one blood sample is collected within the first 12 months of treatment, especially within the first 3 months.

5. The use according to claim 2 or 3 wherein the at least one blood sample is collected within the first 30 days of treatment.

6. Use of Imatinib or a pharmaceutically acceptable salt thereof for the manufacture of a medicament for the treatment of a Ph+ leukemia, wherein
(a) a predetermined fixed amount of Imatinib or a pharmaceutically acceptable salt thereof is administered to the human patient suffering from a Ph+ leukemia,
(b) at least one blood sample from said patient is collected within the first 12 months of treatment,
(c) the plasma trough level (Cmin) of Imatinib is determined, and
(d) the dose of Imatinib or a pharmaceutically acceptable salt thereof is adjusted in a manner that a Cmin of at least about 800 ng/mL of Imatinib is achieved in said patient.

7. The use according to claim 6 wherein the dose of the pharmaceutically acceptable salt of Imatinib is adjusted in a manner that a Cmin between about 1000 and about 3000 ng/mL of Imatinib is achieved in said patient.

8. The use according to claim 6 or 7 wherein the Ph+ leukemia is Philadelphia chromosome positive acute lymphoblastic leukemia (Ph+ ALL) or chronic myeloid leukemia (CML).

9. Use of Imatinib or a pharmaceutically acceptable salt thereof for the manufacture of a medicament for the treatment of a Ph+ leukemia, wherein
(a) a predetermined fixed amount of Imatinib or a pharmaceutically acceptable salt thereof is administered to the human patient suffering from a Ph+ leukemia,
(b) at least one blood sample from said patient is collected within the first 12 months of treatment,
(c) the plasma trough level (Cmin) of N-{5-[4-(piperazino-methyl)-benzoylamido]-2-methylphenyl}-4-(3-pyridyl)-2-pyrimidine-amine is determined, and
(d) the dose of Imatinib or a pharmaceutically acceptable salt thereof is adjusted in a manner that a Cmin of at least about 150 ng/mL of N-{5-[4-(piperazino-methyl)-benzoylamido]-2-methylphenyl}-4-(3-pyridyl)-2-pyrimidine-amine is achieved in said patient.

10. The use according to claim 9 wherein the dose of the pharmaceutically acceptable salt of Imatinib is adjusted in a manner that a Cmin between about 250 and about 700 ng/mL of N-{5-[4-(piperazino-methyl)-benzoylamido]-2-methylphenyl}-4-(3-pyridyl)-2-pyrimidine-amine is achieved in said patient.

11. The use according to any one of claims 1 to 10 wherein the mono-mesylate salt of Imatinib is administered.

12. The use according to claim 11 wherein in step (a) a daily dose of between about 200 and about 800 mg of the mono-mesylate salt of Imatinib is administered orally.

13. The use according to claim 11 wherein in step (a) a daily dose of about 400 mg of the mono-mesylate salt of Imatinib is administered orally.

14. The use according to any one of claims according to any one of claims 1 to 13, wherein the dose adjustment is only applied to patients having an intermediate Sokal score (ISS).
